# EUROPEAN PATENT SPECIFICATION

(11) **EP 1 601 406 B1**
(45) Date of publication and mention of the grant of the patent: **20.05.2009**
(21) Application number: 04737286.7
(22) Date of filing: 01.03.2004
(51) Int. Cl.: A61M 25/01

(54) **APPARATUS FOR REDUCING EXPOSURE TO AN IMAGING BEAM**
ANORDNUNG ZUR VERMINDERUNG DER ABBILDUNGSSTRAHLENBELASTUNG
PROCEDE ET APPAREIL POUR REDUIRE L'EXPOSITION A UN FAISCEAU DE FORMATION D'IMAGES

(30) Priority: 03.03.2003 US 376618
(43) Date of publication of application: 07.12.2005
(73) Proprietor: Murphy, Kieran P., Maryland, Baltimore 222312 (US)
(72) Inventor: Murphy, Kieran P., Maryland, Baltimore 222312 (US)
(74) Representative: Rupp, Christian
(86) International application number: PCT/IB2004/002108
(87) International publication number: WO 2004/078227

(56) References cited:
- US-A- 5 857 997
- US-A- 6 126 654
- US-B2- 6 511 471

## Description

### FIELD OF THE INVENTION

The present invention relates to generally to medical imaging and more particularly relates to a method and apparatus for reducing exposure to an imaging beam such as an X-ray or the like.

### BACKGROUND OF THE INVENTION

Microcatheters introduced through a guiding catheter via the femoral artery are well known and can be used to navigate into the patient's torso or head, and be equipped with different types of tips, according to the procedure being performed. For example, such a microcatheter may be up to one meter or even one-and-a-half meters long and may be equipped to assist in the treatment of an aneurysm in the patient's brain. Due to the length of these microcatheters, enormous skill is required on the part of the surgeon introducing the microcatheter-especially since the target area in the patient's brain may be less than five millimeters in diameter. Typically, the surgeon will blindly introduce a large portion of the microcatheter through the guiding catheter, with only a general idea of where the distal tip of the microcatheter is located at any given time within the patient. The final length of the microcatheter is then introduced under image guidance, such as using a series of X-ray pictures, to determine exactly where the distal tip of the catheter is located in relation to the target area in the patient's brain. This can expose the patient to an undesirable number of X-ray doses. Also, if the surgeon "guesses" incorrectly, it is possible that the surgeon will overshoot the target area before relying on image guidance, and thereby possibly leading to patient injury.
U.S. Patent No. 6,511,471 discloses a system and method for delivering a drug to a target site within a patient's body. The system and method include a steerable guide catheter and a drug delivery catheter. The steerable guide catheter has a first extension tube and a second extension tube that are joined together and form a shoulder. The delivery catheter has a distal docking segment and a proximal docking segment. The guide catheter is inserted into the patient's body, then the delivery catheter is inserted into the guide catheter. The distal docking segment engages the first extension tube, the proximal docking segment engages the second extension tube, and the shoulder limits the distance the delivery catheter can be inserted into the guide catheter. Also, once the delivery catheter is inserted it can be rotated to attach the helical tip to the target site. The guide catheter also includes a steering mechanism as well as a friction mechanism which controls the tension on the steering mechanism. The delivery catheter also includes two luer fittings, each having its own lumen.

### SUMMARY OF THE INVENTION

It is therefore an object of the present invention to provide a novel method and apparatus for reducing exposure to an imaging beam that obviates or mitigates at least one of the above-identified disadvantages of the prior art.
In a first aspect of the invention there is provided an apparatus for reducing exposure to an imaging beam comprising a guiding catheter having a distal tip for insertion to a target area and a proximal tip. The apparatus also comprises a first microcatheter for insertion into the guiding catheter's proximal tip, the first microcatheter having a distal tip that includes a device for treating a condition corresponding to the target area. The apparatus also comprises a locating means operably associated with at least one of the guiding catheter and the first microcatheter for indicating when the distal tip of the microcathether is about exit the distal tip of the guiding catheter at a predetermined point during the insertion.

In the implementation of the first aspect, the first microcatheter is hollow and the device is a second microcatheter for insertion into a proximal tip of the first microcatheter and operable to exit the first microcathether's distal tip. The apparatus further includes a second locating means operably associated with the second microcatheter for indicating when the second microcathether is about to exit the distal tip of the first microcathether during insertion of the second microcatheter.

In a particular implementation of the first aspect, the second microcatheter is selected from the group consisting of a microwire and a microcoil. In a particular implementation of the first aspect the device is a stent.

In a particular implementation of the first aspect, the locating means is a graduation disposed on the first microcatheter that lines up with a predetermined location on the guiding catheter to provide a visual indication of when the distal tip of the microcatheter is about exit the distal tip of the guiding catheter. The apparatus can further include a Touhy-Borst adapter releasably-connectable to the guiding catheter. The predetermined location can be the proximal open end of the adapter.

In a particular implementation of the first aspect the locating means is a textured surfaces located on the first microcatheter that lines up with a predetermined location on the guiding catheter to provide a tactile indication of when the distal tip of the microcatheter is about exit the distal tip of the guiding catheter.

In a particular implementation of the first aspect the apparatus further includes a Touhy-Borst adapter releasably-connectable to the guiding catheter, and the locating means is bulge located on the first microcatheter that has an exterior diameter slightly smaller than an interior diameter of the guiding cathether, such that a resistance to insertion is experienced when the bulge enters a proximal end of the adapter to indicate when the distal tip of the microcatheter is about exit the distal tip of the guiding catheter.

In a particular implementation of the first aspect the locating means provides the indication when a distal tip of the microcatheter is at a distance of between about zero millimeters to about thirty millimeters from exiting the distal tip of the guiding catheter. The distance can be from between about two millimeters to about fifteen millimeters. The distance can be from between about five millimeters to about ten millimeters. The distance can be about seven millimeters.

In a second aspect of the invention there is provided an apparatus for reducing exposure to an imaging beam comprising a guiding catheter having a distal tip for insertion to a target area and a proximal tip, and an adapter releasably connectable to the guiding catheter. The adapter has a proximal opening at a first end and a connection means at the opposite end. The connection means is for connection to the guiding catheter's proximal tip. The adapter is made from a clear material and includes an gauge. The apparatus also includes a first microcatheter for insertion into the proximal opening of the guiding catheter, and through the guiding catheter. The first microcatheter has a distal tip that includes a device for treating a condition in the target area when the device exits the guiding catheter's distal tip. The first microcatheter includes a graduation located towards a proximal end of the first microcatheter, such that when the graduation is lined-up with the gauge the distal tip of the microcatheter is at a predefined distance from exiting the guiding catheter's distal tip.

In a particular implementation of the second aspect, the distance is between about zero millimeters to about thirty millimeters. The distance can be between about two millimeters to about fifteen millimeters. The distance can be between about five millimeters to about ten millimeters. In a particular implementation of the second aspect, wherein the distance is about seven millimeters.

The invention can be used in a method of inserting a cathter comprising:
inserting a guiding catheter into an incision in a patient's skin and passing the guiding catheter through one or more blood vessels until a distal tip of the guiding catheter reaches a target area within the patient; and,
inserting a microcatheter into a proximal opening of the guiding catheter until a locating means located on one of the microcatheter and the guiding catheter indicates that a distal tip of the microcather is at a predetermined distance from exiting a distal tip of the guiding catheter.

In a particular implementation of such method, there is provided the additional steps of:
exposing the target area to an imaging beam to determine a location of the distal tip of the microcatheter in relation to the target area;
directing the distal tip of the microcatheter towards the target area using the imaging beam for guidance;
repeating, as necessary, the exposing step and the directing step until the distal tip of the microcatheter is in a desired location in relation to the target area.

The target area can be any treatable location in a patient's body, such as a blood clot in the patient's head. The guiding catheter can be inserted into an incision at any desired or suitable location on a patient's body, such as into the a vein or an artery, such as the femoral or brachial artery or through a vertebral body of the patient.

### Brief Description of the Drawings

The present invention will now be explained, by way of example only, with reference to certain embodiments and the attached Figures in which:
Figure 1 is an isometric view of an apparatus for reducing exposure to an imaging beam;
Figure 2 shows the apparatus of Figure 1 being used by a surgeon on patient;
Figure 3 is an exploded view of various components of the apparatus of Figure 1;
Figure 4 shows the apparatus in Figure 3 with the microcatheter advanced within the guiding catheter; Figure 5 shows the apparatus of Figure 4 with the microcatheter tip adjacent the target area in the patient;
Figure 6 is a partial view of an apparatus in accordance with another embodiment of the invention;
Figure 7 is a partial view of an apparatus in accordance with another embodiment of the invention;
Figure 8 is a partial view of an apparatus in accordance with another embodiment of the invention;
Figure 9 is a partial view of an apparatus in accordance with another embodiment of the invention; and,
Figure 10 is an exploded view of the apparatus shown in Figure 8; and,
Figure 11 is an isometric view of an apparatus for reducing exposure to an imaging beam in accordance with another embodiment of the invention.

### Detailed Description of the Invention

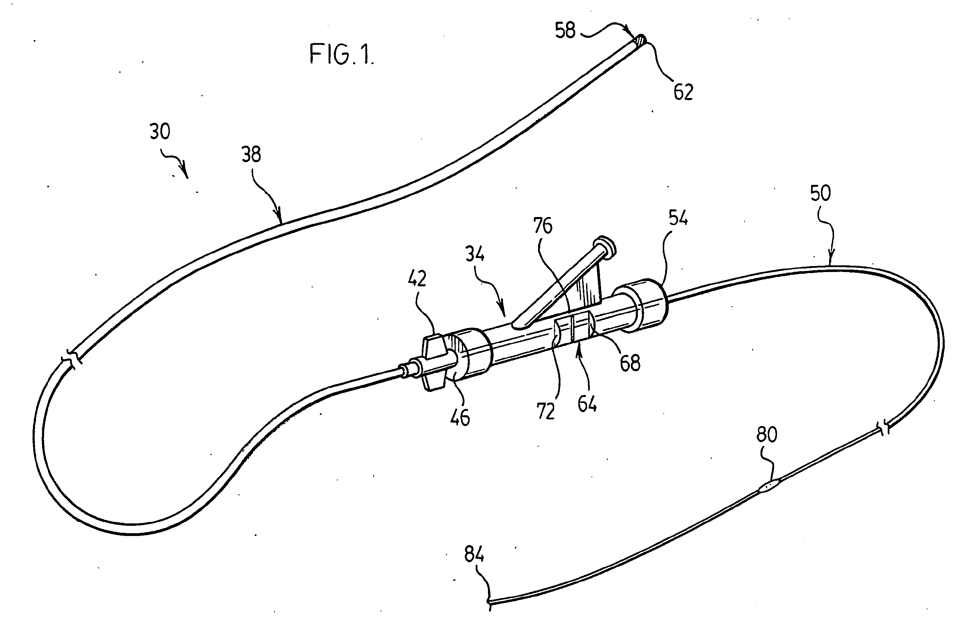

Referring now to Figures 1-5, an apparatus for reducing exposure to an imaging beam is indicated generally at 30. Apparatus 30 comprises a Touhy-Borst adapter 34 releasably-connectable to a guiding catheter 38 via a connector 42 (such as a luer-lock or the like) located at the distal end 46 of adapter 34. Apparatus 30 further comprises a microcathether 50 which is insertable within an open proximal end 54 of adapter 34 and into the lumen of guiding catheter 38.

Guiding catheter 38 is typically made of a flexible material such as silicon, and includes a radioopaque marker 58 at its distal tip 62 that makes the tip of marker 38 visible under an X-ray or other imaging beam under which guiding catheter 38 is intended for use.

In a presently preferred embodiment, adapter 34 is typically made from clear plastic so that microcatheter 50 is visible when being passed through adapter 34. Adapter 34 also includes a gauge 64 that occupies the circumference of a central portion of adapter 34 and is defined by a proximal indicator 68, a distal indicator 72 and a central indicator 76 located half-way between indicators 68 and 72.

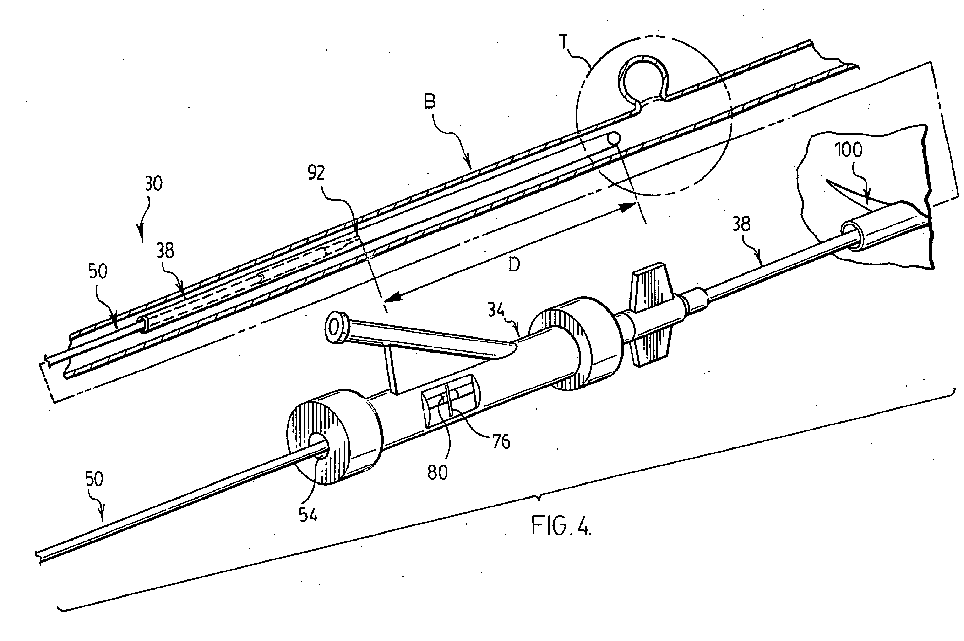

As best seen in Figure 4, microcatheter 50 includes at least one graduation 80 located towards a proximal tip 84 of microcatheter 50, and an angioplasty balloon 88 (which includes a stent) located at the distal tip 92 of microcatheter 50. Graduation 80 is located at a position along microcatheter 50 such that when graduation 80 is aligned with central indicator 76, then tip 92 will be located at a distance (shown in Figure 4 as "D") of about zero millimeters to about thirty millimeters from exiting the tip 62 of guiding catheter 38. More preferably, tip 92 will be located at a distance D of about two millimeters to about fifteen millimeters from exiting the tip 62 of guiding catheter 38. Still more preferably, distance D will be about five millimeters to about ten millimeters. In a presently preferred embodiment however, when graduation 80 is aligned with central indicator 76, then tip 92 will be located at a distance D of about seven millimeters from exiting the tip 62 of guiding catheter 38.

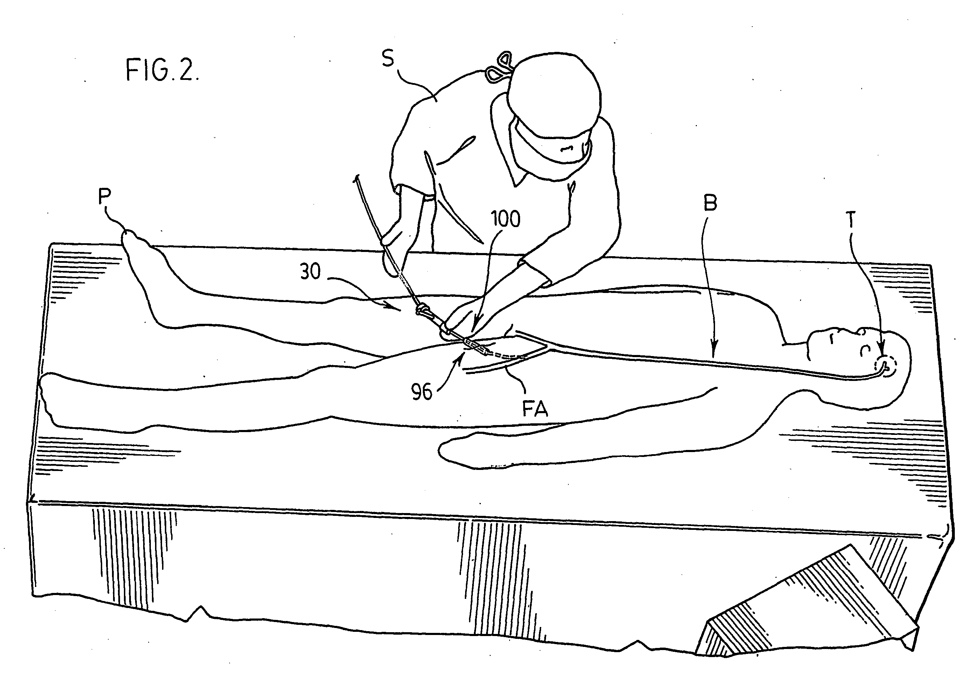

As seen in Figure 2, a surgeon S can use apparatus 30 to treat a patient P. First, surgeon S will make an incision 96 in the thigh to provide access to the femoral artery. Next, surgeon S will place a plastic sleeve 100 in incision 96 to provide an open channel into the femoral artery FA. Surgeon S will then feed guiding catheter 38 into one or more blood vessels B towards a target area T of patient P's brain. In the present example, target area T is an blood clot, but other types and locations of target area T will occur to those of skill in the art. It is to be understood that blood vessels B as shown in Figure 2 are simplified to represent a complex passage of arteries and blood vessels that define a pathway between incision 96 and target area T.

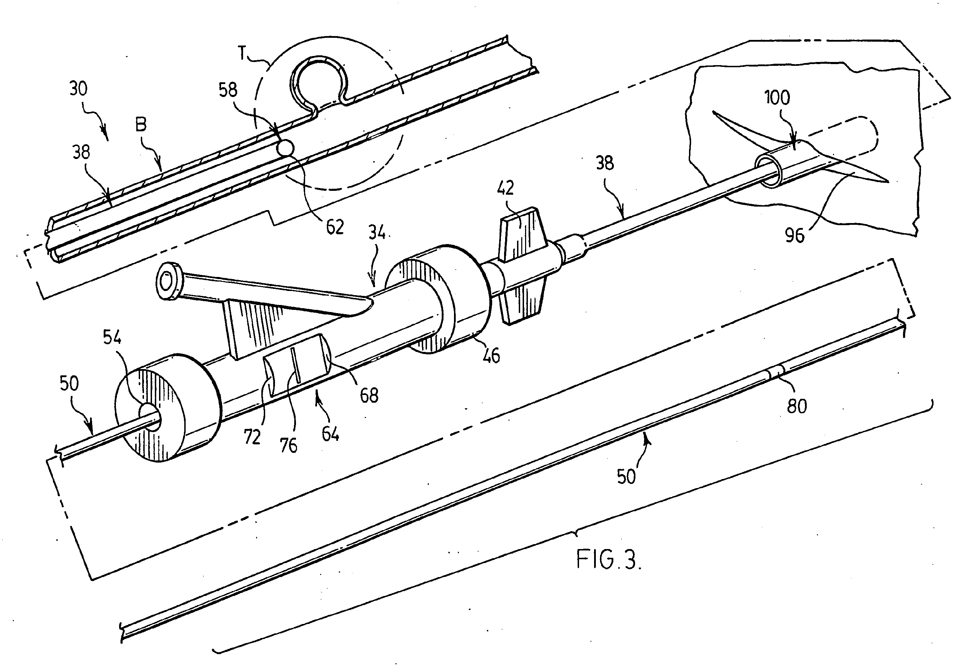

Surgeon S will typically insert guiding catheter 38 under X-ray guidance by taking an X-ray image along artery A at various intervals to determine where tip 62 is located within artery A. Recall that, since tip 62 includes radioopaque marker 58, marker 58 will be visible under X-ray beams. (Note that, in the present embodiment, the entirety of guiding catheter 38 is also radioopaque, but of a different radioopacity than marker 58, to help the surgeon S distinguish between these two parts of catheter 38. In this manner, surgeon S will continue to insert guiding catheter 38 until tip 62 is just proximal to target area T within blood vessel B, as best seen in Figure 3.

As best seen in Figure 3, once guiding catheter 38 is in proper location, distal tip 92 of microcatheter 50 is then inserted into proximal end 54 of adapter 34 and then fed through the lumen of guiding catheter 38 so that distal tip 92 is urged towards tip 62 of guiding catheter 38. During the insertion of microcatheter 50 into guiding catheter 38, surgeon S will watch the position of graduation 80 in relation to proximal end 54. In general, surgeon S may be able to insert microcatheter 50 at a relatively rapid rate prior to the point of graduation 80 actually entering proximal end 54 of adapter 34, and in any event, such insertion need not be performed using X-ray or other image guidance, as the location of graduation 80 is such that an indication is provided that tip 92 remains within the lumen of guiding catheter 38 as long as graduation 80 remains does not pass central indicator 76 of gauge 64.

Thus, as best seen in Figures 3 and 4, once graduation 80 enters proximal end 54 of adapter 34, surgeon S will use additional care as surgeon S continues to insert microcatheter 50, watching carefully as graduation 80 approaches central indicator 76, and finally ceasing further insertion once graduation 80 is actually aligned with central indicator 76. The alignment of graduation 80 with central indicator 76 is shown in Figure 4, which also shows tip 92 located a predefined distance of about seven millimeters from exiting tip 62 of guiding catheter 38.

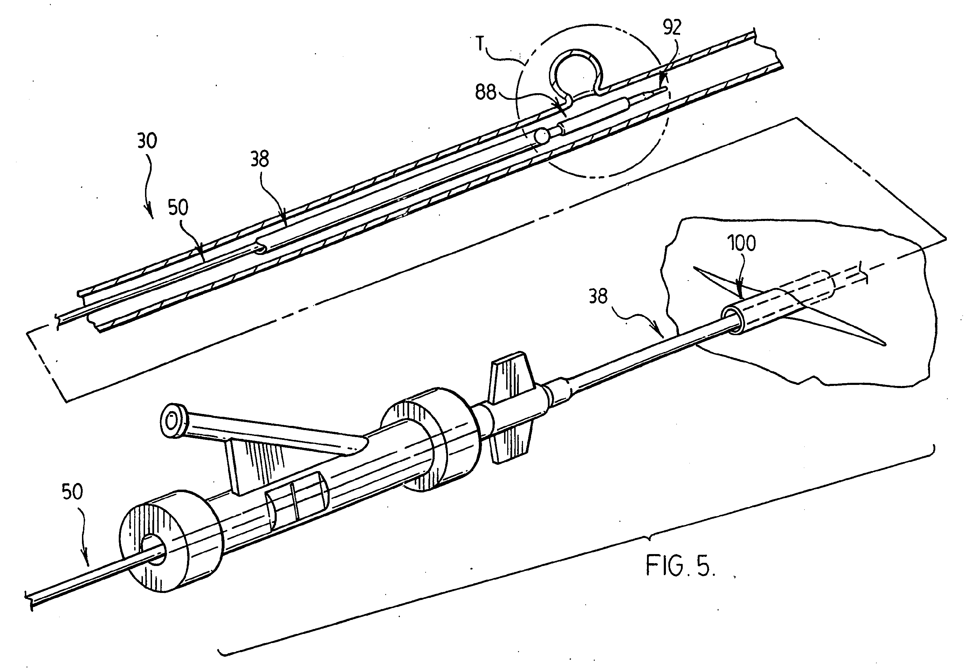

Surgeon S will then continue to slowly insert microcatheter 50 within guiding catheter 38, but will now rely on X-ray guidance to provide an image of where tip 92 is located in relation to target area T. As best shown in Figure 5, such insertion under X-ray guidance thus continues until tip 92 and angioplasty balloon 88 are in a desired position in relation to target area T. Having located tip 92 in the desired location, target area T can then be treated in the usual manner.

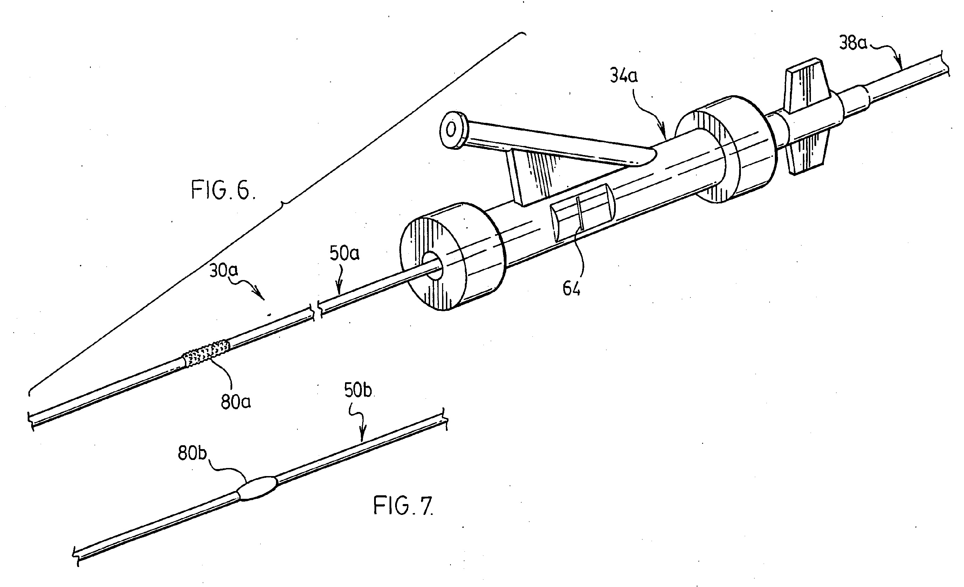

It is to be understood that in other embodiments of the invention graduation 80 could also be other types of locating means or *indicia* that relies on different sensory perceptions on the part of surgeon S. For example, as shown in Figure 6, such locating indicium could be a textured surface 80a in lieu of graduation 80 (but could also be used in addition to graduation 80). Textured surface 80a us located on the exterior of microcatheter 50a. Such textured surface could allow surgeon S to use sensory feedback to feel where microcatheter 50a is located in relation to adapter 34a by means of a change in resistence experienced by the surgeon S when inserting the microcatheter. Textured surface 80a could be used in lieu of, or in addition to graduation 80, and thereby obviate the need for gauge 64. Accordingly, while adapter 34a of Figure 6 includes gauge 64 it is to be understood that gauge 64 can be excluded from the persent embodiment.

Furthermore, other locating means could include a combination of textured surfaces could be applied to the interior of adapter 34a and the exterior of microcatheter 50a, such that surgeon S experiences resistance when that textured surface of microcatheter 50 passes the textured interior of microcatheter 50. Additionally, as shown in, in Figure 7 a bulge 80b can used in lieu of, or in addition to graduation 80 and/or textured surface 80a. Bulge 80b is slightly smaller in diameter than the proximal end 54b of adapter 34b (not shown in Figure 7), such that a small amount of resistance is experienced by surgeon S when bulge 80b enters proximal end 54b. Still further types of locating *indicium* will now occur to those of skill in the art.

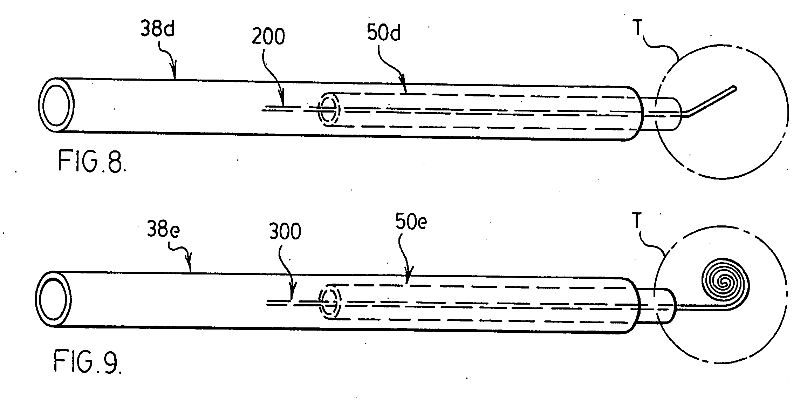

It is to be understood that in other embodiments of the invention, other arrangements of guiding catheters and microcatheters can be constructed. For example, apparatus 30 can be varied such that microcatheter 50 is itself a hollow tube, such that an additional cathether can be inserted within microcatheter 50. The additional catheter would typically include its own graduation or marker at its proximal end that would be located at a position such that when the graduation entered the opening in the proximal end of microcatheter 50, then the additional catheter would be a known distance from exiting tip 92 of the microcatheter 50. By the same token, tip 92 of microcatheter 50 need not have an angioplasty balloon 88, but could include any device for treating a corresponding condition associated with target area T. For example, tip 92 could be characterized by a flexible helical coil, or a bent wire. These variations are shown in Figures 8, 9 and 10. As seen in Figures 8 there is shown a guiding catheter 38d, which telescopically receives a hollow microcatheter 50d therein, and which in turn telescopically receives a microwire 200. The distal tip of microwire 200 is hockey-stick shaped, but is straightened during travel through microcathther 50d. By the same token, in Figure 9, there is shown a guiding catheter 38e, which telescopically receives a hollow microcatheter 50e therein, and which in turn telescopically receives a microcoil 300 that has coiled upon its exit from the distal tip of microcatheter 50e.

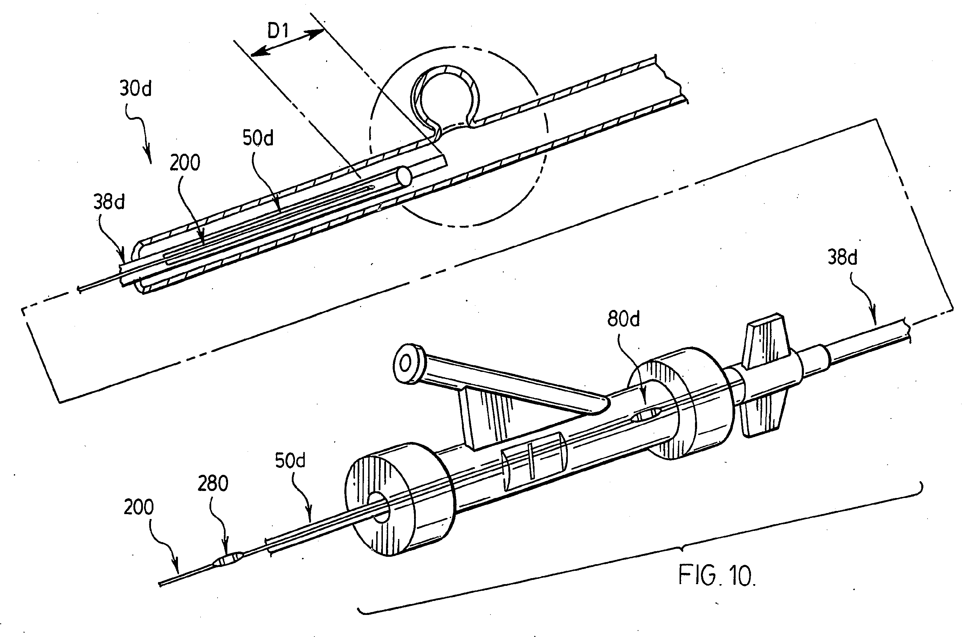

As best seen in Figure 10, microcathether 50d of Figure 8 includes a graduation 80d or other *indicium* to indicate when the distal tip thereof is about to exit from the distal tip of guiding catheter 38d, in substantially the same way graduation 80 on apparatus 30 is configured. However, in addition, the proximal end of microwire 200 also includes a graduation 280, (or other *indicium*), to indicate to surgeon S when the distal tip of microwire 200 is about to exit the distal tip of its respective microcatheter 50d. In the present embodiment, graduation 280 is located to indicate a given distance D1 (where D1 is indicated on Figure 10) when graduation 280 is about to enter the proximal end of microcatheter 50d. Graduation 280 is used to indicate when the distal tip of microwire 200 is of a given distance D1 (where D1 is indicated on Figure 10) of about five mm from exiting the distal tip of its respective microcathether 50d or 50e. Graduation 280 can also be placed to indicate a distance D1 of about seven millimeters, or about ten millimeters, or about fifteen millimeters, as desired. In will now be apparent that configuration in Figure 10 can be also applied to microcoil 300 of Figure 9.

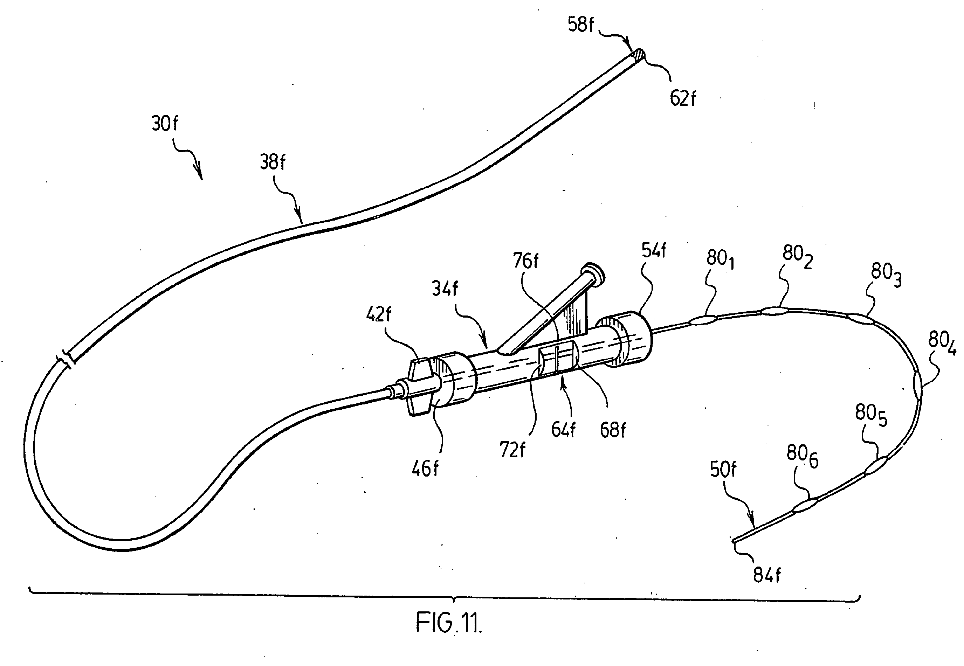

Referring now to Figure 11, an apparatus for reducing exposure to an imaging beam in accordance with another embodiment of the invention is indicated generally at 30f. Like components in apparatus 30f to components of apparatus 30 in Figure 1 have the same reference numeral, but followed by the suffix "f". Thus, apparatus 30f is substantially the same as apparatus 30, except that apparatus 30f includes a plurality of graduations indicated by reference numerals 80₁, 80₂, 80₃, 80₄, 80₅, 80₆. Graduations 80₁, 80₂, 80₃, 80₄, 80₅, 80₆ are placed along the length of microcatheter 50f at a given distance from the distal tip 92f of microcatheter 50f, and accordingly, when inserted into a guiding catheter 38f, each graduation 80₁, 80₂, 80₃, 80₄, 80₅, 80₆ will indicate a different distance that microcatheter 50f has been inserted within guiding catheter 38f. Table I shows a list of presently preferred locations for each graduation 80₁, 80₂, 80₃, 80₄, 80₅, 80₆ and the distances that are represented thereby. (Note that Figure 11 is not drawn to scale.)

**Table I**

| OVERALL LENGTH IN CM OF MICROCATHETER 50_{F} | DISTANCE OF GRADUATION FROM DISTAL TIP 92F OF MICROCATHETER 50F | | | | | |
|---|---|---|---|---|---|---|
| | GRADUATION | | | | | |
| | 80₁ | 80₂ | 80₃ | 80₄ | 80₅ | 80₆ |
| 155 | 15 | 30 | 60 | 90 | 120 | 150 |
| 125 | 20 | 40 | 60 | 80 | 100 | 120 |
| 105 | 5 | 20 | 40 | 60 | 80 | 100 |

From examining Table I, it will now be apparent that microcathethers 50f of different lengths can be interchangeably used with a plurality of different guiding catheters 38f each having different lengths, and that graduations 80₁, 80₂, 80₃, 80₄, 80₅, 80₆ can thus be used to allow an operator to know how far a particular microcatheter 50f has been inserted into a particular guiding catheter 38f, and thereby derive the approximate distance of how far the distal tip of that particular microcatheter 50f is from exiting the distal tip of that particular guiding catheter 38f. For example, assume that a microcatheter 50f of an overall length of one-hundred-and-fifty-five centimeters marked with graduations 80₁, 80₂, 80₃, 80₄, 80₅, 80₆ as shown in Table I is being used. Further assume that a guiding catheter 38f coupled with adapter 34f has an overall length of one-hundred-and-forty centimeters. Further assume that central indicator 76f of the guiding catheter 38f is located five centimeters from the proximal opening 54f of adapter 34f. Table II shows the relative distance between the distal tip 92f of microcatheter 50f to the point of exiting the distal tip 62f of guiding catheter 38f as each graduation 80₁, 80₂, 80₃, 80₄, 80₅, 80₆ lines up with respective central indicator 76f.

**Table II**

| 155 CM MICROCATHETER 50_{F} FROM TABLE I | GRADUATION | | | | | |
|---|---|---|---|---|---|---|
| | 80₁ | 80₂ | 80₃ | 80₄ | 80₅ | 80₆ |
| DISTANCE IN CM OF DISTAL TIP 92F OF MICROCATHETER 50F FROM EXITING DISTAL DIP 62F OF GUIDING CATHETER 38F IN CM THAT MICROCATHETER 50F HAS BEEN INSERTED INTO GUIDING CATHETER 38F | 130 | 105 | 75 | 45 | 15 | -15 (MICROCATHER TIP IS 15CM PAST GUIDING CATHETER TIP) |

It will thus now be apparent that microcatheter 50f can be used with guiding catheters 38f of different lengths. It will also be now apparent that multiple graduations can be used on microcoils and/or microwires that run through guiding catheter 38f.
It will now be apparent that any number of graduations can be used, fewer or greater than the six discussed above in Table I, as desired.

While only specific combinations of the various features and components of the present invention have been discussed herein, it will be apparent to those of skill in the art that desired subsets of the disclosed features and components and/or alternative combinations of these features and components can be utilized, as desired. For example, in the embodiment shown in Figures 1-5, guiding catheter 38 could be varied to include a graduation or marker of its own located near connector 42 which would represent an approximate length that guiding catheter 38 had been inserted within blood vessel B.

It is also to be understood that other types of locating indicia can be used, other than the specific graduation 80 of microcatheter 50 coupled with gauge 64 of adapter 34. For example gauge 64 can be eliminated altogether, and graduation 80 can be located further towards the proximal end of microcatheter 50, such that the position of tip 92 as shown in Figure 4 is reflected by the point at which graduation 80 actually enters proximal end 54 of adapter 54.

Furthermore, graduation 80 is described herein as simply being a visual identifying mark located on the shaft of microcatheter 50 that distinguishes that portion of microcatheter 50 from the remainder of microcatheter 50. However, such an identifying mark could be made in colour to make it easier to view.

The embodiments discussed herein refer to having a single graduation 80 along microcatheter 50, however, it is to be understood that a plurality of graduations 80 could be used along the length of microcatheter 50 to represent different positions of tip 92 in relation to its exit from tip 62 of guiding catheter 38.

The above-described embodiments of the invention are intended to be examples of the present invention and alterations and modifications may be effected thereto, by those of skill in the art, without departing from the scope of the invention which is defined solely by the claims appended hereto.

## Claims

1. An apparatus (30) for reducing exposure to an imaging beam, comprising:
a guiding catheter (38) having a distal tip (62) for insertion to a target area and a proximal tip (68);
a first microcatheter (50) for insertion into said guiding catheter's (38) proximal tip (68),
wherein said first microcatheter (50) has a distal tip (92) that includes a device for treating a condition corresponding to said target area;
wherein said first microcatheter (50) is hollow, and wherein said device is a second microcatheter for insertion into a proximal tip (84) of said first microcatheter (50) and operable to exit said first microcathether's distal tip (92);
a locating means (80) operably associated with at least one of said guiding catheter (38) and said first microcatheter (50) for indicating when said distal tip (92) of said microcathether (50) is about to exit said distal tip (62) of said guiding catheter (38) at a predetermined point during said insertion, and
a second locating means operably associated with said second microcatheter for indicating when said second microcathether is about to exit said distal tip (92) of said first microcathether (50) during insertion of said second microcatheter.

2. The apparatus (30) according to claim 2, wherein said second microcatheter is selected from the group consisting of a microwire (200) and a microcoil (300).

3. The apparatus (30) according to claim 1, wherein said device is a stent (88).

4. The apparatus (30) according to claim 1, wherein said locating means is a graduation (80) disposed on said first microcatheter (50) that lines up with a predetermined location on said guiding catheter (38) to provide a visual indication when said distal tip (92) of said microcatheter (50) is about to exit said distal tip (62) of said guiding catheter (38).

5. The apparatus (30) according to claim 4, wherein said apparatus (30) further includes a Touhy-Borst adapter (34) releasably-connectable to said guiding catheter (38) and said predetermined location is a proximal open end (54) of said adapter (34).

6. The apparatus (30) according to claim 4, wherein said apparatus (30) further includes a Touhy-Borst adapter (34) releasably-connectable to said guiding catheter (38), said adapter (34) is made from a substantially transparent material to allow viewing of said microcatheter (50) passing therethrough, and said predetermined location is an indicator mark located on said adapter (34).

7. The apparatus (30) according to claim 1, wherein said locating means is a textured surfaces located on said first microcatheter (50) that lines up with a predetermined location on said guiding catheter (38) to provide a tactile indication of when said distal tip (92) of said microcatheter (50) is about exit said distal tip (62) of said guiding catheter (38).

8. The apparatus (30) according to claim 1 , wherein said apparatus (30) further includes a Touhy-Borst adapter (34) releasably-connectable to said guiding catheter (38), and
said locating means is a bulge (80b) located on said first microcatheter (50) that has an exterior diameter slightly smaller than an interior diameter of said guiding catheter (38), such that a resistance to insertion is experienced when said bulge (80b) enters a proximal end of said adapter (34) to indicate when said distal tip (92) of said microcatheter (50) is about to exit said distal tip (62) of said guiding catheter (38).

9. The apparatus (30) according to claim 1 , wherein said locating means provides said indication when a distal tip (92) of said microcatheter (50) is at a distance of between about zero millimeters to about thirty millimeters from exiting said distal tip (62) of said guiding catheter (38).

10. The apparatus (30) according to claim 9, wherein said distance is between about two millimeters to about fifteen millimeters.

11. The apparatus (30) according to claim 9 , wherein said distance is between about five millimeters to about ten millimeters.

12. The apparatus (30) according to claim 9, wherein said distance is about seven millimeters.

13. The apparatus (30) of claim 1,
an adapter (34) releasably connectable to said guiding catheter (38),
said adapter (34) having a proximal opening at a first end and a connection means at the opposite end, said connection means for connection to said guiding catheter's (38) proximal tip (68),
said adapter (34) being made from a clear material and including a gauge;
wherein said first microcatheter (50) is inserted into said proximal opening and through said guiding catheter (38).

14. The apparatus (30) according to claim 13, wherein said target area is a predetermined location inside a patient and said guiding catheter (38) is inserted into either one of a vein, an artery or a vertebral body of said patient.

## Patentansprüche

1. Anordnung (30) zur Verminderung der Gefährdung gegenüber einem Abbildungsstrahl, welche umfasst:
einen Führungskatheter (38), der eine distale Spitze (62) zur Einführung in einen Zielbereich und eine proximale Spitze (68) hat;
einen ersten Mikrokatheter (50) zum Einführen in die proximale Spitze (68) dieses Führungskatheters (38),
wobei der erste Mikrokatheter (50) eine distale Spitze (92) hat, welche eine Einrichtung umfasst, um einen Zustand, der dem Zielbereich entspricht, zu behandeln,
wobei der erste Mikrokatheter (50) hohl ist, und
wobei die Einrichtung ein zweiter Mikrokatheter zur Einführung in die proximale Spitze (84) des ersten Mikrokatheters (50) ist und betriebsfähig ist, die distale Spitze (92) des ersten Mikrokatheters zu verlassen;
eine Lokalisierungseinrichtung (80), welche im Betrieb mit zumindest einem von Führungskatheter (38) und ersten Mikrokatheter (50) verbunden ist, um zu zeigen, wenn die distale Spitze (92) des Mikrokatheters (50) im Begriff ist, die distale Spitze (62) des Führungskatheters (38) an einem vorher festgelegten Punkt während der Einführung zu verlassen, und
eine zweite Lokalisierungseinrichtung, welche im Betrieb mit dem zweiten Mikrokatheter verbunden ist, um zu zeigen, wenn der zweite Mikrokatheter im Begriff ist, die distale Spitze (92) des ersten Mikrokatheters (50) während der Einführung des zweiten Mikrokatheters zu verlassen.

2. Anordnung (30) nach Anspruch 2, wobei der zweite Mikrokatheter aus der Gruppe ausgewählt wird, welche aus der Gruppe besteht, welche aus einem Mikrodraht (200) und einer Mikrospule (30) besteht.

3. Anordnung (30) nach Anspruch 1, wobei die Einrichtung eine Stützeinrichtung (88) ist.

4. Anordnung (30) nach Anspruch 1, wobei die Lokalisierungseinrichtung eine Abstufungseinrichtung (80) ist, welche auf dem ersten Mikrokatheter (50) angeordnet ist, welche in einer Linie angeordnet ist mit einer vorher festgelegten Lage auf dem Führungskatheter (38), um eine optische Anzeige zu liefern, wenn die distale Spitze (92) des Mikrokatheters (50) im Begriff ist, die distale Spitze (62) des Führungskatheters (38) zu verlassen.

5. Anordnung (30) nach Anspruch 4, wobei die Anordnung (30) außerdem einen Touhy-Borst-Adapter (34) aufweist, der lösbar mit dem Führungskatheter (38) verbindbar ist, und die vorher festgelegte Lage ein proximales offenes Ende (54) des Adapters (34) ist.

6. Anordnung (30) nach Anspruch 4, wobei die Anordnung (30) außerdem einen Touhy-Borst-Adapter (34) aufweist, der lösbar mit dem Führungskatheter (38) verbindbar ist, wobei der Adapter (34) aus einem im Wesentlichen transparenten Material hergestellt ist, um das Betrachten des Mikrokatheters (50), der **dadurch** läuft, zuzulassen, und die vorher festgelegte Lage eine Indikatormarkierung ist, welche auf dem Adapter (34) angeordnet ist.

7. Anordnung (30) nach Anspruch 1, wobei die Lokalisierungseinrichtung eine strukturierte Fläche ist, welche auf dem ersten Mikrokatheter (50) angeordnet ist, welche in Linie angeordnet ist mit einer vorher festgelegten Lage auf dem Führungskatheter (38), um eine tastbare Indikation davon zu liefern, wenn die distale Spitze (92) des Mikrokatheters (50) im Begriff ist, die distale Spitze (62) des Führungskatheters (38) zu verlassen.

8. Anordnung (30) nach Anspruch 1, wobei die Anordnung (30) außerdem einen Touhy-Borst-Adapter (349 aufweist, der mit dem Führungskatheter (38) lösbar verbindbar ist, und
die Lokalisierungseinrichtung ein Wulst (80b) ist, der auf dem ersten Mikrokatheter (50) angeordnet ist, der einen äußeren Durchmesser hat, der etwas kleiner ist als ein Innendurchmesser des Führungskatheters (38), so dass ein Widerstand hinsichtlich der Einführung erfahren wird, wenn der Wulst (80b) ein proximales Endes des Adapters (34) betritt, um zu zeigen, wenn die distale Spitze (92) des Mikrokatheters (50) im Begriff ist, die distale Spitze (62) des Führungskatheters (38) zu verlassen.

9. Anordnung (30) nach Anspruch 1, wobei die Lokalisierungseinrichtung die Anzeige liefert, wenn eine distale Spitze (92) des Mikrokatheters (50) in einem Abstand von zwischen ungefähr 10 mm bis ungefähr 30 mm vom Verlassen der distalen Spitze (62) des Führungskatheters (38) ist.

10. Anordnung (30) nach Anspruch 9, wobei der Abstand zwischen ungefähr 2 mm bis ungefähr 15 mm ist.

11. Anordnung (30) nach Anspruch 9, wobei der Abstand zwischen ungefähr 5 mm bis ungefähr 10 mm ist.

12. Anordnung (30) nach Anspruch 9, wobei der Abstand ungefähr 7 mm ist.

13. Anordnung (30) nach Anspruch 1,
wobei ein Adapter (34) lösbar mit dem Führungskatheter (38) verbindbar ist, wobei der Adapter (34) eine proximale Öffnung an einem ersten Ende und eine Verbindungseinrichtung an dem abgewandten Ende hat, wobei die Verbindungseinrichtung zum Verbinden mit der proximalen Spitze (68) des Führungskatheters (38) dient,
wobei der Adapter (34) aus einem transparenten Material hergestellt ist und ein Eichmaß umfasst;
wobei der erste Mikrokatheter (50) in die proximale Öffnung und durch den Führungskatheter (38) eingeführt wird.

14. Anordnung (30) nach Anspruch 13, wobei der Zielbereich eine vorher festgelegte Lage innerhalb eines Patienten ist, und der Führungskatheter (38) in eines von einer Vene, einer Arterie oder einem Wirbelkörper des Patienten eingeführt wird.

## Revendications

1. Appareil (30) destiné à réduire l'exposition à un faisceau de formation d'image, comprenant :
un cathéter de guidage (38), présentant une pointe distale (62), destiné à être inséré dans une zone cible et une pointe proximale (68) ;
un premier microcathéter (50) destiné à être inséré à l'intérieur de ladite pointe proximale (68) du cathéter de guidage (38),
dans lequel ledit premier microcathéter (50) présente une pointe distale (92) qui comporte un dispositif destiné à traiter un état correspondant à ladite zone cible ;
dans lequel ledit premier microcathéter (50) est creux ; et
dans lequel ledit dispositif est un second microcathéter destiné à être inséré à l'intérieur d'une pointe proximale (84) dudit premier microcathéter (50) et est capable de fonctionner pour quitter ladite pointe distale (92) du premier microcathéter ;
des moyens de positionnement (80) associés de manière opérationnelle à au moins un parmi ledit cathéter de guidage (38) et ledit premier microcathéter (50) destinés à indiquer le moment où ladite pointe distale (92) dudit microcathéter (50) est sur le point de quitter ladite pointe distale (62) dudit cathéter de guidage (38) en un point prédéterminé pendant ladite insertion, et
des seconds moyens de positionnement associés de manière opérationnelle au dit second microcathéter destinés à indiquer le moment où ledit second microcathéter est sur le point de quitter ladite pointe distale (92) dudit premier microcathéter (50) pendant l'insertion dudit second microcathéter.

2. Appareil (30) selon la revendication 2, dans lequel ledit second microcathéter est sélectionné parmi le groupe constitué par un microfil (200) et une microbobine (300).

3. Appareil (30) selon la revendication 1, dans lequel ledit dispositif est une endoprothèse vasculaire (88).

4. Appareil (30) selon la revendication 1, dans lequel lesdits moyens de positionnement sont une graduation (80) disposée sur ledit premier microcathéter (50) qui s'aligne sur un emplacement prédéterminé sur ledit cathéter de guidage (38) pour fournir une indication visuelle du moment où ladite pointe distale (92) dudit microcathéter (50) est sur le point de quitter ladite pointe distale (62) dudit cathéter de guidage (38).

5. Appareil (30) selon la revendication 4, dans lequel ledit appareil (30) comporte en outre un adaptateur Touhy-Borst (34) pouvant être raccordé de manière libérable audit cathéter de guidage (38) et ledit emplacement prédéterminé est une extrémité ouverte proximale (54) dudit adaptateur (34).

6. Appareil (30) selon la revendication 4, dans lequel ledit appareil (30) comporte en outre un adaptateur Touhy-Borst (34) pouvant être raccordé de manière libérable audit cathéter de guidage (38), ledit adaptateur (34) est fabriqué à partir d'un matériau sensiblement transparent pour permettre de visualiser ledit microcathéter (50) le traversant, et ledit emplacement prédéterminé est une marque indicatrice positionnée sur ledit adaptateur (34).

7. Appareil (30) selon la revendication 1, dans lequel lesdits moyens de positionnement sont une surface texturée positionnée sur ledit premier microcathéter (50) qui s'aligne sur un emplacement prédéterminé sur ledit cathéter de guidage (38) pour fournir une indication tactile du moment où ladite pointe distale (92) dudit microcathéter (50) est sur le point de quitter ladite pointe distale (62) dudit cathéter de guidage (38).

8. Appareil (30) selon la revendication 1, dans lequel ledit appareil (30) comporte en outre un adaptateur Touhy-Borst (34) pouvant être raccordé de manière libérable au dit cathéter de guidage (38), et
lesdits moyens de positionnement sont un renflement (80b) positionné sur ledit premier microcathéter (50) qui présente un diamètre extérieur légèrement inférieur à un diamètre intérieur dudit cathéter de guidage (38), de telle sorte qu'une résistance à l'insertion est expérimentée lorsque ledit renflement (80b) pénètre dans une extrémité proximale dudit adaptateur (34) pour indiquer le moment où ladite pointe distale (92) dudit microcathéter (50) est sur le point de quitter ladite pointe distale (62) dudit cathéter de guidage (38).

9. Appareil (30) selon la revendication 1, dans lequel lesdits moyens de positionnement fournissent ladite indication du moment où une pointe distale (92) dudit microcathéter (50) doit encore parcourir une distance comprise entre zéro millimètre et environ trente millimètres pour quitter ladite pointe distale (62) dudit cathéter de guidage (38).

10. Appareil (30) selon la revendication 9, dans lequel ladite distance est comprise entre environ deux millimètres et environ quinze millimètres.

11. Appareil (30) selon la revendication 9, dans lequel ladite distance est comprise entre environ cinq millimètres et environ dix millimètres.

12. Appareil (30) selon la revendication 9, dans lequel ladite distance mesure environ sept millimètres.

13. Appareil (30) selon la revendication 1,
un adaptateur (34) pouvant être raccordé de manière libérable au dit cathéter de guidage (38),
ledit adaptateur (34) présentant une ouverture proximale à une première extrémité et des moyens de raccordement à l'extrémité opposée, lesdits moyens de raccordement étant destinés à le raccorder à ladite pointe proximale (68) du cathéter de guidage (38),
ledit adaptateur (34) étant fabriqué à partir d'un matériau transparent et comportant une jauge ;
dans lequel ledit premier microcathéter (50) est inséré à l'intérieur de ladite ouverture proximale et à travers ledit cathéter de guidage (38).

14. Appareil (30) selon la revendication 13, dans lequel ladite zone cible est un emplacement prédéterminé à l'intérieur d'un patient et ledit cathéter de guidage (38) est inséré à l'intérieur d'une veine, d'une artère ou d'un corps vertébral dudit patient.
